# EUROPEAN PATENT SPECIFICATION

(11) **EP 1 985 225 B1**
(45) Date of publication and mention of the grant of the patent: **08.12.2010**
(21) Application number: 08007637.5
(22) Date of filing: 18.04.2008
(51) Int. Cl.: A61B 1/00, A61B 1/005

(54) **Medical apparatus**
Medizinisches Gerät
Appareil médical

(30) Priority: 26.04.2007 JP 2007117489
(43) Date of publication of application: 29.10.2008
(73) Proprietor: Olympus Medical Systems Corp., Tokyo 151-0072 (JP)
(72) Inventor: Ito, Jin, Tokyo 192-8512 (JP); Masaki, Yutaka, Tokyo 192-8512 (JP)
(74) Representative: von Hellfeld, Axel

(56) References cited:
- EP-A- 1 623 664
- WO-A-2006/059721
- WO-A-2006/059722
- JP-A- 2006 116 131
- US-A- 4 474 419
- US-A1- 2002 098 732
- US-A1- 2006 287 575

## Description

The present invention relates to a medical apparatus comprising an endoscope body, which is disposed, for example, at a proximal end of an insertion section of an endoscope, and a power unit to which the endoscope body is connected.

In an endoscope serving as a medical apparatus, a body unit is provided on a proximal end portion of an insertion section which is inserted into a body cavity. In the insertion section, a distal-end rigid section is provided at a distal end of an elongated flexible tube section via a bending section. A universal cord, which is connected to, e.g. a light source device, is connected to the body unit. Built-in components, such as a light guide fiber of an illumination optical system, a signal line which is connected to an image pick-up element of an observation optical system, bending operation wires, and air-feed/water-feed conduits or a suction conduit, are inserted in the insertion section. The body unit is provided with connection sections of the light guide fiber of the illumination optical system, the signal line and various conduits. Further, the body unit is provided with a bending operation section for bend-operating the bending section, and various operation buttons for air feed, water feed and suction.

As regards the endoscope having the above-described structure, there is known an endoscope in which built-in components inserted in the insertion section and built-in components inserted in the universal cord are attachable/detachable in the body unit. Specifically, there is known an endoscope in which connectors for an illumination optical system and a signal line are provided in the vicinity of the body unit of the endoscope, and the illumination optical system and signal line are detachably connected via these connectors at the body unit (see, e.g. Jpn. Pat. Appln. KOKAI Publication No. H10-14867 (patent document 1)).

There is also known an endoscope apparatus in which a connection section is provided at a proximal end portion of an insertion section, and the proximal end portion of the insertion section is detachably connected to a body unit. In this case, a plurality of kinds of insertion sections are selectively connectable to a single body unit (see, e.g. Jpn. Pat. Appln. KOKAI Publication No. 2006-116131 (patent document 2)). In this endoscope apparatus, a connection section between the insertion section and the body unit is provided with connectors of an illumination optical system, a signal line and various conduits for air feed, water feed and suction. When the insertion section and the body unit are connected, these built-in components are configured to be connected at the same time.

A motor-driven bending type endoscope apparatus is also disclosed, wherein a connection section is provided at a proximal end portion of an insertion section, and the proximal end portion of the insertion section is detachably connected to a body unit. In this apparatus, an electric motor is built in the body unit. A motor-driven angle mechanism is driven by a driving force of an electric motor, thereby bending a bending section (see, e.g. Jpn. Pat. Appln. KOKAI Publication No. 2006-288751 (patent document 3) and Jpn. Pat. Appln. KOKAI Publication No. 2002-224016 (patent document 4)). In this apparatus, too, a connection section between the insertion section and the body unit is provided with connectors of an illumination optical system, a signal line and various conduits for air feed, water feed and suction. When the insertion section and the body unit are connected, these built-in components are configured to be connected at the same time.

In the above-mentioned patent documents 3 and 4, two electric motors (an electric motor for UD (for an up-and-down direction) and an electric motor for RL (for a right-and-left direction)) are built in a bend-driving unit for driving the bending section. The rotation of the rotational shafts of these motors is transmitted to clutches via deceleration gears, and respective bending drums are rotated. By the rotation of the bending drums, the associated angle wires are pulled, and thus the bending section is bent in four directions, namely, in upward, downward, leftward and rightward directions.

In patent document 1, the endoscope is configured such that connectors for an illumination optical system and a signal line are provided in the vicinity of the body unit of the endoscope, and the illumination optical system and signal line on the body unit side are detachably connected. However, various conduits for air feed, water feed and suction are also built in the insertion section and the body unit. Thus, since the connection section of the illumination optical system and electrical system needs to be shut off from the fluid system for air feed, water feed and suction, the internal structure of the connection section becomes complex.

In patent document 2, the endoscope apparatus is configured such that the connection section of the illumination optical system and electrical system and the connection section of various conduits of the fluid system are connected at the same time by connecting the insertion section and the body unit. These connections are effected within a single connector housing. Thus, like patent document 1, the internal structure of the connection section becomes complex.

In patent documents 3 and 4, the driving force transmission system from the electric motors, which constitute the bend-driving unit, is exposed. Thus, a water-proof measure is required for the bend-driving unit when cleaning is performed, and the structure is complex. Further, when the endoscope is used, there is a case in which a torsion force in a direction about the axis of the insertion section may act on the insertion section. In this case, since the torsion force of the insertion section is directly applied to the driving force transmission section, the driving force transmission system may possibly be adversely affected.

WO 2006/059721 A1 and WO 2006/059722 A1 both describe an electrically bendable endoscope comprising an insertion section body which is releasably connectable to a motor unit by inserting a portion of the insertion section body into a reception chamber formed in the motor unit. A fluid connection unit is releasably connectable to the insertion section body and is adapted to be received in a corresponding recess formed in the motor unit when the fluid connection unit is connected to the insertion section body. The reception chamber formed in the motor unit and the portion of the insertion section body which is inserted into the reception chamber when the motor unit and the insertion section body are connected both are box-shaped and have a substantially rectangular cross-section.

US 2006/0287575 A1 discloses an endoscope comprising a concave AWS adapter attachment section which is adapted to be connected to a corresponding AWS adapter.

EP 1 623 664 A1 relates to an endoscope system, wherein a light source is provide with a general connector receptacle portion to which a plurality of devices can be releasably connected.

JP 2006 116131 A describes an endoscope system comprising an endoscope body which is connectable to either to a first or a second insertion part.

The present invention has been made in consideration of the above-described circumstances, and the object of the invention is to provide a medical apparatus such as an endoscope, wherein a fluid connection unit for fluid connection of, e.g. air feed and water feed, can be shut off, by a simple structure, from a non-fluid connection unit for non-fluid connection of, e.g. an illumination optical system, a signal line and a driving force system, and the reliability of a water-proof measure is high.

This problem is solved by a medical apparatus as defined in claim 1.

According to an aspect of the present invention, there is provided a medical apparatus comprising: a first member which constitutes a part of the medical apparatus; a second member having an outer peripheral portion which is surrounded by walls, and having a reception chamber to which the first member is detachably connected; a fluid connection unit which is connected to the first member on an outside of the reception chamber; and a non-fluid connection unit which is connected to the first member on an inside of the reception chamber.

When the first member and the second member are connected, both the fluid connection unit and the non-fluid connection unit are substantially simultaneously connected.

The reception chamber has peripheral walls with a rectangular cross-sectional shape, a circular cross-sectional shape, or a combination of a rectangular cross-sectional shape and a circular cross-sectional shape.

The peripheral walls include a rectangular portion with a rectangular cross-sectional shape on an entrance side in an attachment/detachment direction of the first member, and a circular portion with a circular cross-sectional shape on a depth side.

Preferably, the fluid connection unit is a fluid connection connector of at least one of an air-feed conduit, a water-feed conduit and a forward water-feed conduit.

Preferably, the non-fluid connection unit is a connection connector of at least one of light, electricity and driving force.

Preferably, a driving-side member for driving force transmission is provided on a side wall of the reception chamber, and the first member is provided with a driven-side member which is connected to the driving-side member when the first member is inserted in the reception chamber.

A light transmission connector receiver is provided on an end face of the reception chamber, and the first member is provided with a light transmission connector which is connected to the light transmission connector receiver when the first member is inserted in the reception chamber.

An electric signal connector receiver is provided on a side wall of the reception chamber, and the first member is provided with an electric signal connector which is connected to the electric signal connector receiver when the first member is inserted in the reception chamber.

Preferably, the first member is an endoscope body having an insertion section which is inserted into a body cavity, and the second member is a power unit which executes control of an endoscope.

Preferably, the power unit includes a connection section for connection to the endoscope body, to which at least one of an optical system, an electrical system and a driving force system is connected.

Preferably, the endoscope body includes a fluid connection connector receiver of at least one of an air-feed conduit, a water-feed conduit and a forward water-feed conduit, and the power unit is provided with a fluid conduit connector on an outside of the reception chamber, the fluid conduit connector being connected to the fluid connection connector receiver.

Preferably, the endoscope body includes a bending section which is provided in the insertion section, and a driven-side member which is interlocked with the bending section, the power unit includes, in a reception chamber in which the endoscope body is received, a driving-side member of a bend-driving mechanism which is driven by an electric motor, and the driving-side member and the driven-side member are connected when the endoscope body is received in the reception chamber.

Preferably, the endoscope body includes a bending section which is provided in the insertion section, and a driven-side member which is interlocked with the bending section, the power unit includes, in a reception chamber in which the endoscope body is received, a driving-side member which is driven by an operation handle, and the driving-side member and the driven-side member are connected when the endoscope body is received in the reception chamber.

According to the present invention, the non-fluid connection unit for the optical, electrical and driving force systems is provided within the reception chamber that is surrounded by the peripheral walls, and the fluid connection unit for the air-feed, water-feed and forward water-feed conduits is provided outside the reception chamber. Thereby, fluid connection can be shut off from non-fluid connection, no fluid enters the non-fluid connection unit for the optical, electrical and driving force systems, and the reliability can be enhanced.

The invention can be more fully understood from the following detailed description when taken in conjunction with the accompanying drawings, in which:
FIG. 1 is a perspective view showing the entire structure of a medical apparatus according to a first embodiment of the present invention;
FIG. 2 is a block diagram that schematically shows the medical apparatus according to the first embodiment;
FIG. 3 is a perspective view showing a state before an endoscope body and a power unit according to the first embodiment are connected;
FIG. 4 is a longitudinal cross-sectional view showing an internal structure of an insertion-section-side body of the endoscope according to the first embodiment;
FIG. 5 is a cross-sectional view taken along line V-V in FIG. 4;
FIG. 6 is a cross-sectional view taken along line VI-VI in FIG. 5;
FIG. 7 is a partially cross-sectional plan view of a support structure of a main shaft of the insertion-section-side body of the endoscope according to the first embodiment;
FIG. 8 is a transverse cross-sectional view showing an internal structure of a power unit of the endoscope according to the first embodiment;
FIG. 9 is a transverse cross-sectional view that shows, in partial cross-section, an internal structure of the power unit of the endoscope according to the first embodiment;
FIG. 10 is a side view of a driving section of the power unit of the endoscope according to the first embodiment;
FIG. 11 is a cross-sectional view of the driving section of the power unit of the endoscope according to the first embodiment;
FIG. 12 is a transverse cross-sectional view of the power unit of the endoscope according to the first embodiment;
FIG. 13 is a perspective view showing a part of an insertion-section-side body of an endoscope according to a second embodiment of the invention;
FIG. 14 is a perspective view showing an insertion-section-side body of an endoscope;
FIG. 15 is a perspective view showing an insertion-section-side body of an endoscope;
FIG. 16A is a transverse cross-sectional view showing a driving section of a power unit of an endoscope according to a further embodiment of the invention; and FIG. 16B is a side view showing the driving section of the power unit of the endoscope according to the fifth embodiment.

Embodiments of the present invention will now be described with reference to the accompanying drawings.

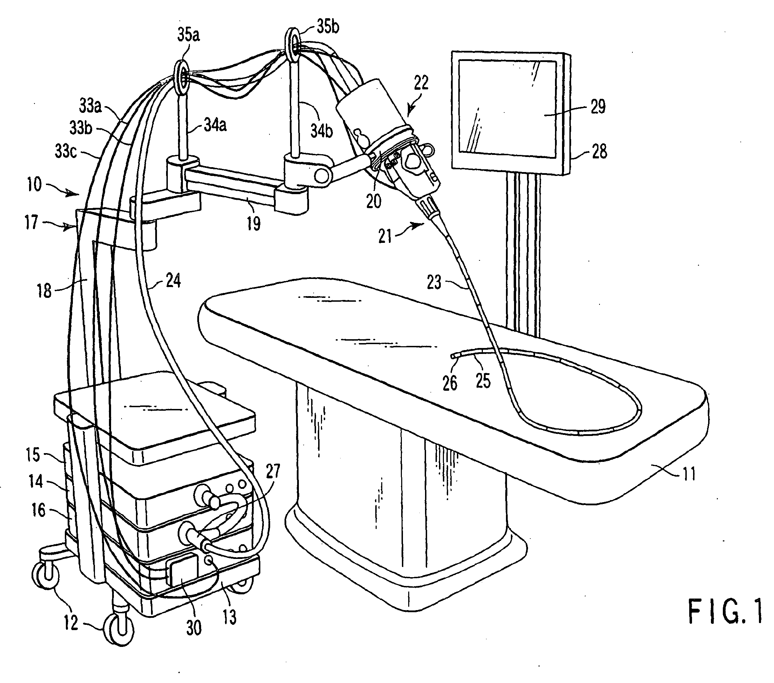

FIG. 1 to FIG. 12 show a first embodiment of the invention. FIG. 1 shows the entire structure of a medical apparatus 1. FIG. 1 is a perspective view showing a state in which the medical apparatus 10 is disposed on the side of an operation bed 11. The medical apparatus 10 is supported on a support table 13 that includes casters 12. A light source device 14, a video processor 15 and a control box 16 are mounted on the support table 13. A support column 18, which constitutes a support mechanism 17, is vertically erectingly provided on the support table 13. An upper end portion of the support column 18 is provided with an endoscope holding section 20 as a medical device holding section via a plurality of arms 19 which are rotatable in a horizontal plane.

An endoscope 21 serving as a medical device is held by the endoscope holding section 20. The endoscope 21 includes a body unit 22, an elongated insertion section 23, and a universal cord 24. The body unit 22 is held by the endoscope holding section 20. A proximal end portion of the insertion section 23 is connected to the body unit 22. The insertion section 23 is provided with a bending section 25 and a distal-end structure section 26. One end portion.of the universal cord 24 is connected to the body unit 22. The other end portion of the universal cord 24 is provided with a connector 27. The connector 27 is connected to the light source device 14. A light guide 46 and a signal cable 48, which are to be described later, are inserted in the universal cord 24.

The distal-end structure section 26 of the endoscope 21 includes, at its distal end face, an illumination lens of an illumination optical system (not shown) and an observation lens of an observation optical system (not shown). A light guide 46 of an optical fiber, which guides illumination light, is continuously inserted in the universal cord 24, body unit 22 and insertion section 23. Illumination light from the light source device 14 is emitted from the illumination lens at the front face of the distal-end structure section 26 via the light guide 46 that is inserted through the universal cord 24, body unit 22 and insertion section 23. Thereby, the inside of a body cavity is illuminated.

The observation optical system is provided with an image pick-up unit 47, such as a solid image pick-up element, at a position of a focusing plane of the observation lens. An observation image of the observation lens, which observes the inside of the body cavity, is converted to a video signal by the image pick-up unit 47. A signal cable 48, which transmits a video signal from the image pick-up unit 47, is continuously inserted through the insertion section 23, body unit 22 and universal cord 24. The video signal from the image pick-up unit 47 is transmitted to the video processor 15 via the signal cable 48. The video signal is subjected to a predetermined signal process by the video processor 15.

A control panel 28 is disposed on the operation bed 11. The control panel 28 is provided with a display unit 29 and an operation section, such as a touch-panel operation section, on a display screen of the display unit 29. The video signal that is output from the video processor 15 is transmitted to the control panel 28. A predetermined endoscopic image is displayed on the display unit 29 of the control panel 28. A surgeon can input various operational instructions from the operation section of the control panel 28.

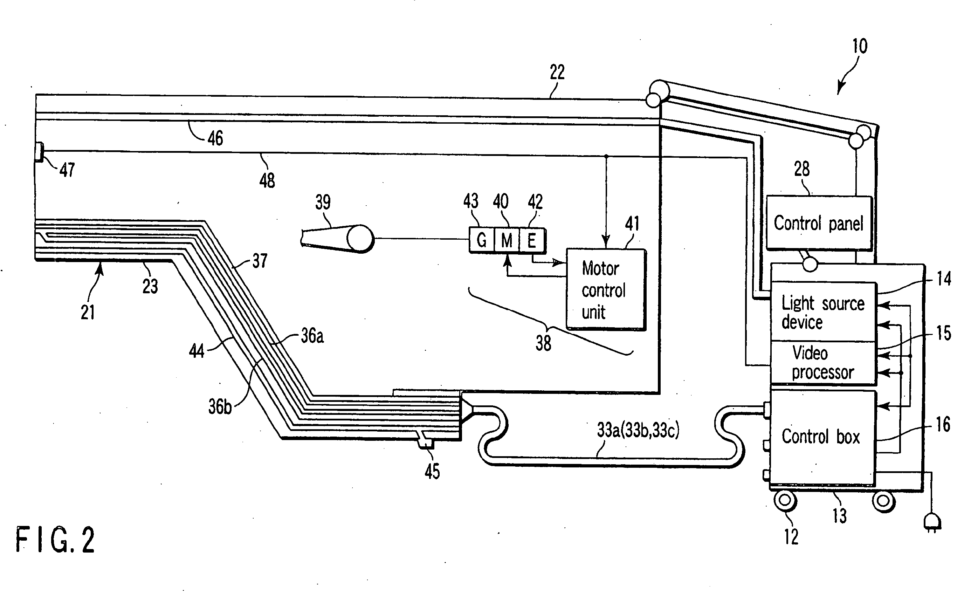

The control box 16 functions to operate an electromagnetic valve unit (not shown) for performing air feed and water feed at a time of using the endoscope 21 for observation and medical treatment. The electromagnetic valve unit executes control of air-feed/water-feed and a suction operation via an air-feed/water-feed conduit and a suction conduit, which are provided within the insertion section 23. As shown in FIG. 2, a system controller of the control box 16 is electrically connected to the light source device 14 and video processor 15.

A fluid control cassette 30 is detachably attached to the electromagnetic valve unit of the control box 16 shown in FIG. 1. The fluid control cassette 30 includes a flow amount adjusting mechanism having valve bodies which are associated with air feed, water feed and forward water feed. The electromagnetic valve unit drives the flow amount adjusting mechanism of the fluid control cassette 30.

One end of each of three tubes 33a, 33b and 33c for performing air feed, water feed and forward water feed to the endoscope 21 is connected to the control box 16. The other end of each of the three tubes 33a, 33b and 33c is connected to the body unit 22 of the endoscope 21. These tubes 33a, 33b and 33c are formed of hollow flexible resin materials.

A plurality of support rods 34a and 34b are erectingly provided on the arms 19 of the support mechanism 17. The support rods 34a and 34b are provided with retainers 35a and 35b which support intermediate portions of the tubes 33a, 33b and 33c and the universal cord 24.

FIG. 2 is a structural view that schematically shows internal structures of the body unit 22 and insertion section 23 of the endoscope 21. The distal-end structure section 26 of the insertion section 23 is provided with an air-feed/water-feed conduit 36 and a forward water-feed conduit 37. A proximal end portion of the air-feed/water-feed conduit 36 is divided into two branch conduits. One of the branch conduits is coupled to a distal end portion of an air-feed conduit 36a, and the other branch conduit is coupled to a distal end portion of a water-feed conduit 36b.

The air-feed conduit 36a, water-feed conduit 36b and forward water-feed conduit 37 extend toward the body unit 22 through the insertion section 23. Proximal end portions of these three conduits 36a, 36b and 37 are connected to the above-mentioned other ends of the tubes 33a, 33b and 33c. Specifically, the control box 16 is made to communicate with the air-feed/water-feed conduit 36 and forward water-feed conduit 37 of the distal-end structure section 26 of the insertion section 23 via the fluid control cassette 30, tubes 33a, 33b and 33c and three conduits 36a, 36b and 37. Thereby, if the electromagnetic valve unit of the control box 16 is driven and an air-feed/water-feed operation is executed from the fluid control cassette 30, air-feed/water-feed can be performed from the distal-end structure section 26 via the tubes 33a and 33b and air-feed/water-feed conduit 36. In addition, if the electromagnetic valve unit is driven and a forward water-feed operation is executed, forward water-feed can be performed from the distal-end structure section 26 via the forward water-feed conduit 37.

Further, a bend-driving mechanism 38 (to be described later), which is controlled in accordance with a bending operation instruction, is built in the body unit 22 of the endoscope 21. The bend-driving mechanism 38 is configured to bend-operate the bending section 25 in upward, downward, leftward and rightward directions via an angle wire 39. Specifically, the bend-driving mechanism 38 includes an electric motor 40, a motor control unit 41, an encoder 42 and a deceleration gear 43. The electric motor 40 produces a driving force by rotation. The motor control unit 41 executes an overall control of the bend-driving mechanism 38 including the electric motor 40. The encoder 42 generates data representing operation states of, e.g. the rotation speed and rotation amount of the driving shaft of the electric motor 40. The deceleration gear 43 decelerates the rotational driving force of the driving shaft of the electric motor 40. The bend-driving mechanism 38 is bend-driving means which is composed of the electric motor 40 and various members that are formed to transmit and disconnect the driving force that is produced by the electric motor 40. Upon receiving the driving force from the bend-driving mechanism 38, the angle wire 39 is pulled and driven.

A forceps conduit 44 for insertion of a treatment device, such as a forceps, is inserted in the insertion section 23. A distal end portion of the forceps conduit 44 communicates with a forceps hole of the distal-end structure section 26. A proximal end of the forceps conduit 44 communicates with a forceps insertion hole 45 (to be described later) which is formed at a proximal end portion of the insertion section 23 in the vicinity of the body unit 22. Thus, the treatment device, such as a forceps, which is inserted from the forceps insertion hole 45 can be projected from the forceps hole of the distal-end structure section 26 through the forceps conduit 44.

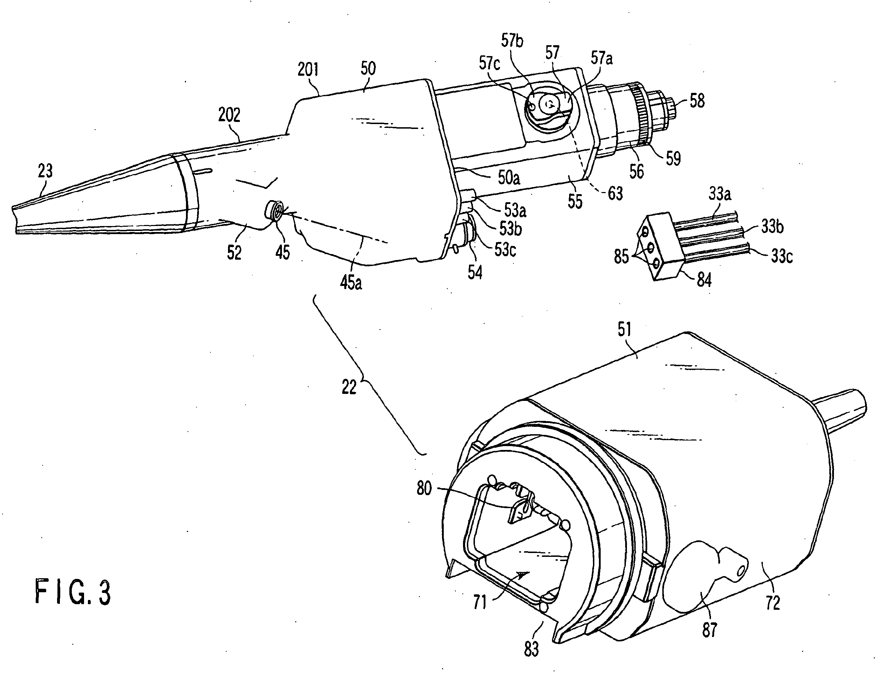

FIG. 3 is a perspective view showing the body unit 22 of the endoscope 21. The body unit 22 comprises an insertion-section-side body 50 as a first member, and a power unit 51 as a second member. The insertion-section-side body 50 is detachably attached to the power unit 51.

To begin with, external structures of the insertion-section-side body 50 and power unit 51 are described. The insertion-section-side body 50 includes a substantially box-shaped casing portion 201 and a circular cylindrical casing portion 202 having a less outside diameter than the box-shaped casing portion 201. A projection portion 52 is provided on an outer peripheral surface of the cylindrical casing portion 202. The projection portion 52 is provided with the forceps insertion hole 45. A center line 45a of the forceps insertion hole 45 is inclined outward, relative to the axial direction of the insertion-section-side body 50. Thus, even if the power unit 51 is attached to the insertion-section-side body 50, the power unit 51 does not hinder the insertion/removal of the treatment device in/from the forceps insertion hole 45.

An attachment surface 50a, which is perpendicular to the axial direction of the insertion-section-side body 50, is provided at an intermediate portion of the insertion-section-side body 50. A fluid connection connector is provided on the attachment surface 50a. The fluid connection connector includes an air-feed mouthpiece 53a, a water-feed mouthpiece 53b, a forward water-feed mouthpiece 53c and a water leak detection mouthpiece 54. The openings of these air-feed mouthpiece 53a, water-feed mouthpiece 53b, forward water-feed mouthpiece 53c and water leak detection mouthpiece 54 are disposed toward the proximal end side. In other words, the air-feed mouthpiece 53a, water-feed mouthpiece 53b and forward water-feed mouthpiece 53c are disposed on the proximal end side of the insertion-section-side body 50, that is, at positions spaced apart from the forceps insertion hole 45.

Since the treatment device, such as a forceps or a catheter, is inserted/removed via the forceps insertion hole 45, there may be a case in which the forceps insertion hole 45 becomes an unclean area. However, since the forceps insertion hole 45 is spaced apart from the air-feed mouthpiece 53a, water-feed mouthpiece 53b and forward water-feed mouthpiece 53c, infection to parts, other than the forceps insertion hole 45, can be prevented.

An endoscope auto-cleaning device, which cleans the endoscope 21 after use, is required to automatically perform three functions, i.e. a function of water leak detection, a function of air feed/water feed to respective conduits, and a function of insertion of a brush to the respective conduits via the air-feed mouthpiece 53a, water-feed mouthpiece 53b and forward water-feed mouthpiece 53c. The air-feed mouthpiece 53a, water-feed mouthpiece 53b, forward water-feed mouthpiece 53c and water leak detection mouthpiece 54 are laterally arranged in line and the opening of the water leak detection mouthpiece 54 is disposed in parallel to the air-feed mouthpiece 53a, water-feed mouthpiece 53b and forward water-feed mouthpiece 53c. Thereby, the endoscope auto-cleaning device can perform the above-described three functions by control in one direction alone. Moreover, the water leak detection mouthpiece 54 is disposed outermost in consideration of preventing other mouthpieces from becoming obstacles when water leak detection is manually performed.

An angular cylindrical portion 55 and a circular cylindrical portion 56 are integrally provided on a proximal end portion of the insertion-section-side body 50. The circular cylindrical portion 56 is disposed at the rear end of the angular cylindrical portion 55. The above-described bend-driving mechanism 38 is built in the angular cylindrical portion 55. A driven-side coupling 57, which functions as a driven-side member that is interlocked with the bend-driving mechanism 38, is provided on an outside part of the angular cylindrical portion 55. A light guide connector 58 is provided on a rear end face of the circular cylindrical portion 56. The light guide connector 58 is a light transmission connector receiver which is connected to the light guide 46. Further, a rear end portion of the circular cylindrical portion 56 is formed of an electrically insulating material, and a great number of electrodes 59 functioning as electric signal connectors are disposed in a circumferential direction on an outer peripheral surface of the rear end portion of the circular cylindrical portion 56.

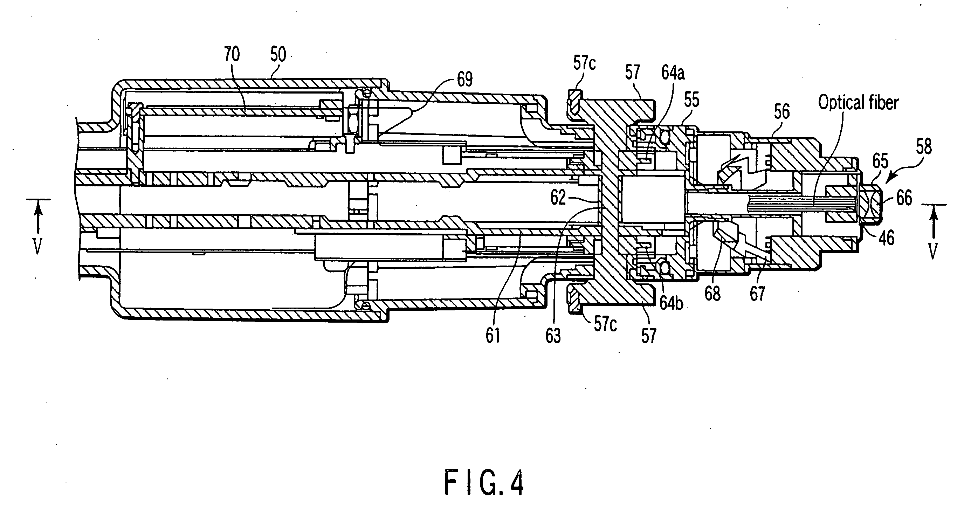

FIG. 4 to FIG. 6 show an internal structure of the insertion-section-side body 50. A chassis 61 having a U-cross section is provided within the insertion-section-side body 50. The chassis 61 is formed of a metallic material with rigidity, such as aluminum. The chassis 61 is disposed in the front-and-rear direction of the insertion-section-side body 50, and the chassis 61 is fixed on the inner wall of the insertion-section-side body 50 and on the inner wall of the angular cylindrical portion 55. The chassis 61 is provided with a bearing 62 in a direction perpendicular to the axial direction of the angular cylindrical portion 55. A main shaft 63 of the driven-side coupling 57, which constitutes the bend-driving mechanism 38, is rotatably supported on the bearing 62.

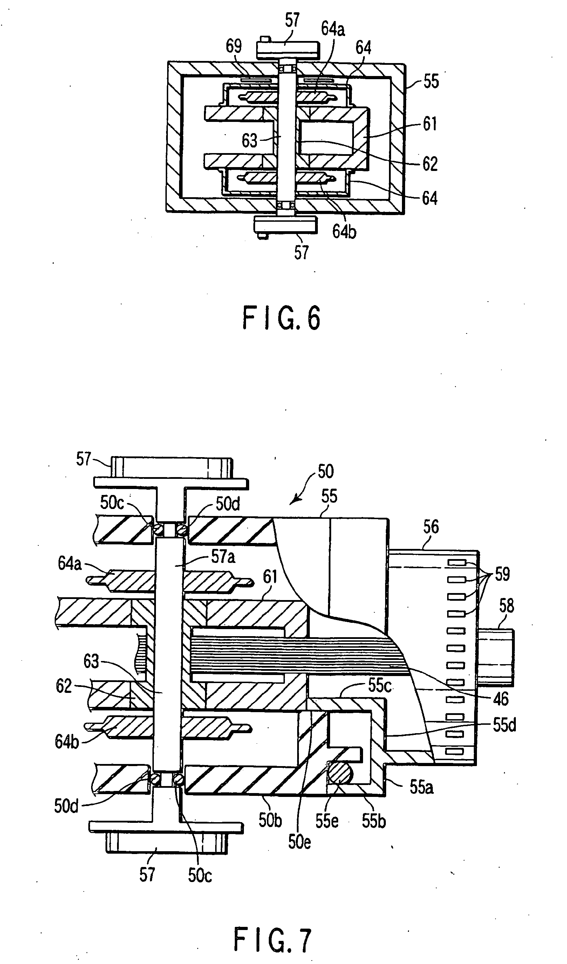

A sprocket 64a for UD (up and down) is provided on an upper-side (in FIG. 4) outer surface of the chassis 61, and a sprocket 64b for RL (right and left) is provided on a lower-side outer surface of the chassis 61. The sprocket 64a for UD and the sprocket 64b for RL are rotatably engaged on the main shaft 63. As shown in FIG. 6, the sprocket 64a for UD and the sprocket 64b for RL are covered with sprocket covers 64. The sprocket covers 64 are fixed to the chassis 61.

As shown in FIG. 6, both end portions of the main shaft 63 project outward from outside walls of the angular cylindrical portion 55. The respective projecting portions are provided with driven-side couplings 57 which are interlocked with the sprocket 64a for UD and the sprocket 64b for RL. As shown in FIG. 3, each driven-side coupling 57 is a rectangular member which is rotatable about the main shaft 63. A narrow-width portion 57a is formed at one end portion of the driven-side coupling 57, and a large-width portion 57b is formed at the other end portion of the driven-side coupling 57. An engaging pin 57c is projectingly provided on the large-width portion 57b.

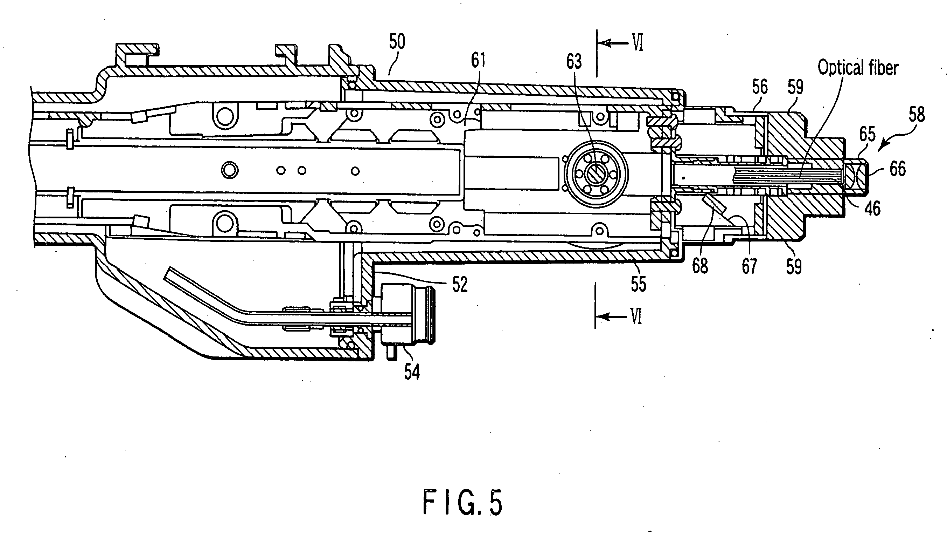

Further, as shown in FIG. 4 and FIG. 5, the light guide 46 is inserted in an axial center part within the circular cylindrical portion 56. The light guide 46 extends continuous with the above-described light guide 46 that is inserted in the insertion section 23 and body unit 22. A lens frame 65 is projected rearward from a rear end portion of the circular cylindrical portion 56. A relay lens 66 is mounted in the lens frame 65. A rear end face of the light guide 46 is coupled to the relay lens 66.

A first flexible board 67 is disposed on the outer periphery of the light guide 46. The electrodes 59 are electrically connected to the first flexible board 67. The first flexible board 67 is provided with a connector 68. One end portion of a second flexible board 69 is connected to the connector 68. As shown in FIG. 6, the second flexible board 69 extends outside the sprocket cover 64 and is guided to the inside of the angular cylindrical portion 55. In addition, the other end portion of the second flexible board 69 is electrically connected to an image pick-up printed board 70 which is provided within the insertion-section-side body 50. The image pick-up printed board 70 is connected to the image pick-up unit, such as a solid image pick-up element, via the signal cable (not shown) that is inserted in the insertion section 23.

An additional description is given of the insertion-section-side body 50. As shown in FIG. 7, a through-hole 50c is provided in an outer wall 50b of the insertion-section-side body 50. The main shaft 63 projects outward through the through-hole 50c. A gap between the main shaft 63 and through-hole 50c is liquid-tightly sealed by an O ring 50d. An alignment member 55a is provided on the rear end face of the outer wall 50b. The alignment member 55a aligns the insertion-section-side body 50 and the power unit 51.

The alignment member 55a is formed of a metallic material with rigidity, such as stainless steel. An outer wall portion 55b, an inner wall portion 55c and a rear-end wall portion 55d are integrally provided on the alignment member 55a. The outer wall portion 55b is formed to have the same outside shape as the outer wall 50b of the angular cylindrical portion 55. The inner wall portion 55c of the alignment member 55a abuts upon an abutment portion 50e of the outer wall 50b and is aligned. Similarly, a rear end portion of the chassis 61 abuts upon the abutment portion 50e of the outer wall 50b and is aligned. Accordingly, the alignment member 55a and the chassis 61 are aligned via the abutment portion 50e of the outer wall 50b of the insertion-section-side body 50. Further, liquid-tight sealing between the rear end face of the outer wall 50b and the outer wall portion 55b is effected by an O ring 55e.

In this manner, in the insertion-section-side body 50, the chassis 61 and the outer wall 50b are aligned with the alignment member 55a being used as a reference. Thus, since the alignment between the insertion-section-side body 50 and the power unit 51 is exactly performed, the driven-side couplings 57 are exactly aligned with the insertion-section-side body 50. Accordingly, when the insertion-section-side body 50 is connected to the power unit 51, alignment between them is also exactly performed. Thereby, the driven-side couplings 57 and driving-side couplings (to be described later) is also exactly carried out, and the dimensional precision of the parts in the vicinity of the couplings and the reliability of the liquid-tight structure can be improved.

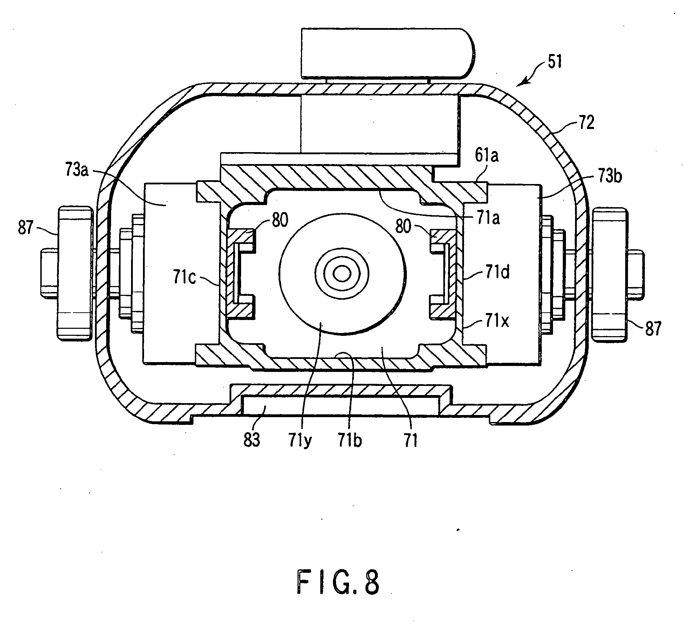

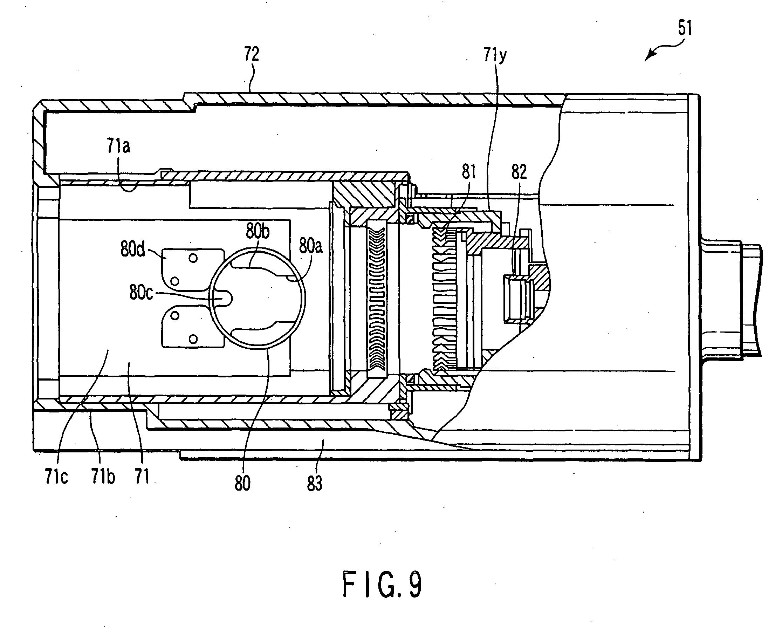

FIG. 8 and FIG. 9 show the internal structure of the power unit 51. As shown in FIG. 8, the power unit 51 is covered with a substantially rectangular casing 72. A reception chamber 71 is provided within the casing 72. The reception chamber 71 is formed of a frame that is formed of a material such as aluminum. The distal end side of the reception chamber 71 is opened.

An angular cylindrical hole portion 71x having a rectangular cross-sectional shape is formed at a distal end side of the reception chamber 71. A circular cylindrical hole portion 71y having a circular cross-sectional shape is formed on a depth side of the reception chamber 71. When the power unit 51 and insertion-section-side body 50 are to be coupled, the angular cylindrical portion 55 and circular cylindrical portion 56 of the insertion-section-side body 50 are engaged in the reception chamber 71. At this time, the angular cylindrical portion 55 of the insertion-section-side body 50 is engaged in the angular cylindrical hole portion 71x of the reception chamber 71, and the circular cylindrical portion 56 is engaged in the circular cylindrical hole portion 71y.

The peripheral walls of the angular cylindrical hole portion 71x are formed of an upper surface 71a, a lower surface 71b, a left side surface 71c and a right side surface 71d. Gently curved R surfaces are formed at four corner portions of the angular cylindrical hole portion 71x. A driving unit 73a for UD, which drives the bend-driving mechanism 38, is provided on an outside surface of the frame, which corresponds to the left side surface 71c. Similarly, a driving unit 73b for RL is provided on an outside surface of the frame, which corresponds to the right side surface 71d.

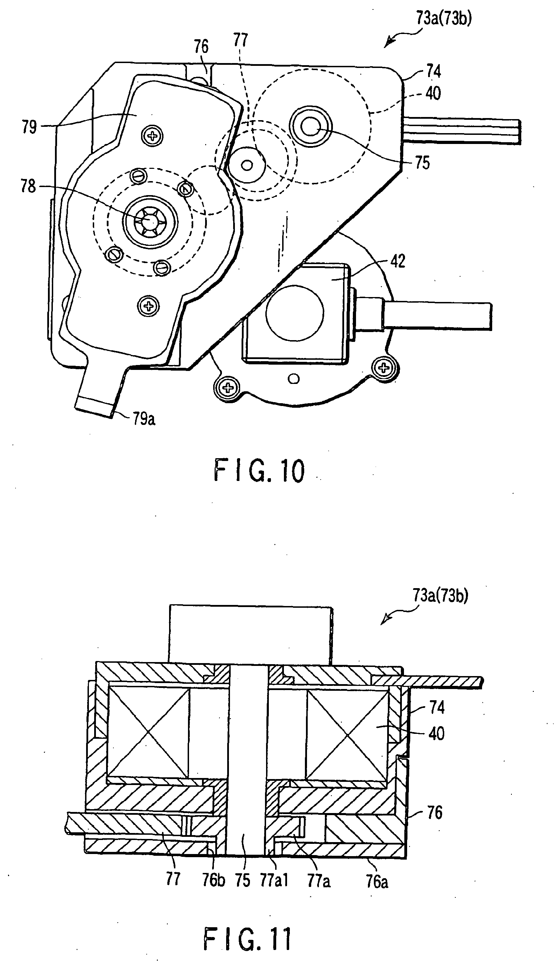

The driving unit 73a for UD and the driving unit 73b for RL have the same structure. FIG. 10 shows the structure of, for instance, the driving unit 73a for UD. The above-described electric motor 40 is provided in a motor case 74. A driving shaft 75 of the electric motor 40 is coupled to a rotational shaft 78 via deceleration gears 77. As shown in FIG. 11, the deceleration gears 77 are covered with a gear case 76. The rotation of the driving shaft 75 of the electric motor 40 is transmitted to the rotational shaft 78 via the deceleration gears 77, and the rotational shaft 78 is rotated in an interlocked state. The deceleration gears 77 are provided with the above-described encoder 42 which detects the rotational angular speed. Electromagnetic clutches (not shown) are provided in the transmission systems of the deceleration gears 77 and rotational shafts 78 of the driving unit 73a for UD and driving unit 73b for RL.

As is shown in FIG. 10, in each of the driving unit 73a for UD and driving unit 73b for RL, the electric motor 40 and encoder 42 are disposed such that they are vertically spaced apart. Further, the rotational shaft 78 of each of the driving unit 73a for UD and driving unit 73b for RL is provided with a cam frame 79 which rotates together with the rotational shaft 78. The driving unit 73a for UD and driving unit 73b for RL are coupled by stays 79a of the cam frames 79.

Further, as shown in FIG. 11, the motor case 74 which accommodates the electric motor 40 is formed of a material such as aluminum. The motor case 74 is coupled to the inner surface of the gear case 76 that is formed of a material such as aluminum. Specifically, such a structure is adopted that the area of heat radiation is increased by the gear case 76 and the heat produced from the electric motor 40 is efficiently radiated.

The gear case 76 is attached to a gear plate 76a which is fixed to a base member within the casing 72. A through-hole 76b is provided in the gear plate 76a. The through-hole 76b is engaged with a boss portion 77al of a motor pinion 77a which is a part of the deceleration gears 77. The through-hole 76b has a greater diameter than the boss portion 77a1, and an opening, which is formed of a gap, is provided therebetween. This opening is closed by attaching each of the driving unit 73a for UD and driving unit 73b for RL to the base member of the reception chamber 71 within the casing 72. Accordingly, since there is no need to use a special closing member, the driving unit 73a for UD and driving unit 73b for RL can be reduced in size.

The rotational shaft 78 of the driving unit 73a for UD and the rotational shaft 78 of the driving unit 73b for RL are disposed to be opposed to each other, with the reception chamber 71 being interposed. As shown in FIG. 8, an inner end portion of the rotational shaft 78 of the driving unit 73a for UD projects to the inside of the reception chamber 71 from the left side surface 71c. Similarly, an inner end portion of the rotational shaft 78 of the driving unit 73b for RL projects to the inside of the reception chamber 71 from the right side surface 71d. Driving-side couplings 80, which are driving-side members, are engaged with the projecting portions of the respective rotational shafts 78.

As shown in FIG. 9, the driving-side coupling 80 is provided with a small-width recess portion 80a, a large-width recess portion 80b and an engaging recess portion 80c. Reference numeral 80d denotes a guide member which guides the driven-side coupling 57 to the driving-side coupling 80. When the driving-side coupling 80 and the driven-side coupling 57 are to be coupled, the small-width portion 57a and large-width portion 57b of the driven-side coupling 57 are engaged with the small-width recess portion 80a and large-width recess portion 80b. Further, the engaging pin 57c is engaged with the engaging recess portion 80c. At this time of engagement, the rotation of the driving-side coupling 80 is transmitted to the driven-side coupling 57.

As shown in FIG. 9, a great number of electric contacts 81 are provided on the inner peripheral surface of the circular cylindrical hole portion 71y of the casing 72. A light guide connector receiving portion 82 is provided on the rear end face of the circular cylindrical hole portion 71y. The electric contacts 81 of the circular cylindrical hole portion 71y are put in contact with, and electrically connected to, the great number of electrodes 59 of the circular cylindrical portion 56 of the insertion-section-side body 50. The light guide connector receiving portion 82 is connected to the light guide connector 58, and light transmission is performed.

Thus, if the angular cylindrical portion 55 and circular cylindrical portion 56 of the insertion-section-side body 50 are engaged in the reception chamber 71 of the power unit 51, the angular cylindrical portion 55 is engaged in the angular cylindrical hole portion 71x and the circular cylindrical portion 56 is engaged in the circular cylindrical hole portion 71y. The driven-side coupling 57 is engaged with the driving-side coupling 80, and the transmission of the driving force system is effected. At the same time, the electrodes 59 come in contact with the electric contacts 81, and the electric system is rendered conductive. Furthermore, the light guide connector 58 is connected to the light guide connector receiving portion 82, and light transmission is effected.

As described above, the reception chamber 71 of the power unit 51 is provided with connection connectors such as the driving-side couplings 80, electric contacts 81 and light guide connector receiving portion 82. The insertion-section-side body 50 is provided with the driven-side couplings 57, electrodes 59 and light guide connector 58. When the insertion-section-side body 50 is connected to the reception chamber 71 of the power unit 51, the driving-side couplings 80 are coupled to the driven-side couplings 57, and at the same time the electric contacts 81 and the electrodes 59 are connected and the light guide connector receiving portion 82 and the light guide connector 58 are connected. Thereby, a non-fluid connection unit is constituted.

In the present embodiment, the driving-side couplings 80 are provided on the side walls of the reception chamber 71. In an alternative structure, the driving-side couplings 80 may be provided on the depth-side wall of the reception chamber 71 and may be engaged with the driven-side couplings 57. Besides, in the present embodiment, the electric contacts 81, which come in contact with the electrodes 59, are provided on the side wall of the reception chamber 71 and the light guide connector receiving portion 82, which is to be connected to the light guide connector 58, is provided on the depth-side wall of the reception chamber 71. Alternatively, the electric contacts 81, which come in contact with the electrodes 59, may be provided on the depth-side wall of the reception chamber 71 and the light guide connector receiving portion 82, which is to be connected to the light guide connector 58, may be provided on the side wall of the reception chamber 71.

As shown in FIG. 9, a recess portion 83 is provided under the lower surface of the casing 72 so as to extend in the axial direction of the power unit 51. A fluid connection connector 84 (see FIG. 3) is received in the recess portion 83. The fluid connection connector 84 is formed of a rubber block. As shown in FIG. 3, the fluid connection connector 84 is provided with three connection ports 85. The three tubes 33a, 33b and 33c for performing air feed, water feed and forward water fee are connected at one end to inner end portions of the three connection ports 85. The air-feed mouthpiece 53a, water-feed mouthpiece 53b and forward water-feed mouthpiece 53c are detachably connected to outer end portions of the three connection ports 85 of the fluid connection connector 84. Thereby, a fluid connection unit is constituted such that fluid connection connectors of air-feed, water-feed and forward water-feed conduits are connected outside the power unit 51.

Accordingly, if the insertion-section-side body 50 and the power unit 51 are connected, the fluid connection connector 84, to which the three tubes 33a, 33b and 33c for air feed, water feed and forward water feed are connected, is connected at the same time to the air-feed mouthpiece 53a, water-feed mouthpiece 53b and forward water-feed mouthpiece 53c of the insertion-section-side body 50. Thus, air feed, water feed and forward water feed from the distal-end structure section 26 of the endoscope 21 are enabled.

Bending operation levers 87 are provided on outside parts of the casing 72. The bending operation levers 87 are coupled, respectively, to the rotational shafts 78 of the driving unit 73a for UD and the driving unit 73b for RL. Thus, with the operation of the bending operation levers 87, the driving unit 73a for UD and the driving unit 73b for RL can manually be operated.

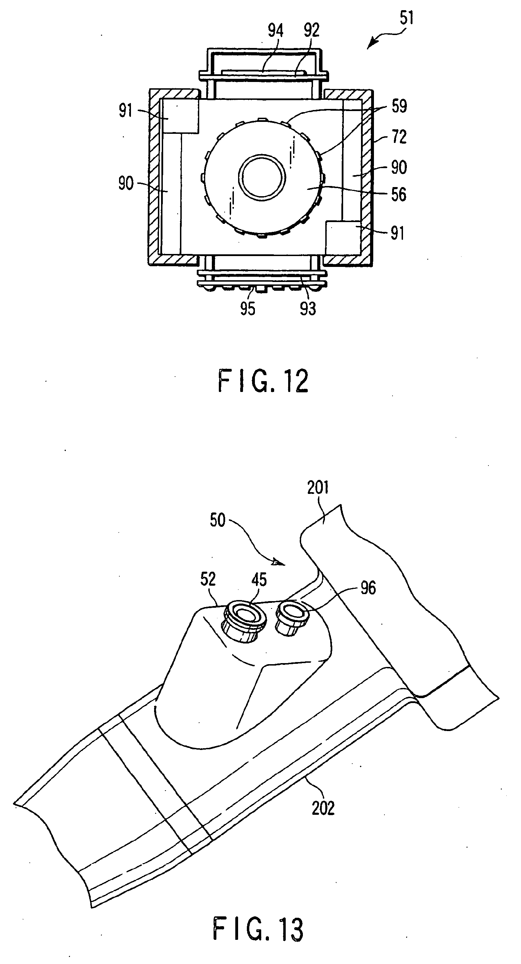

FIG. 12 shows the structure of a main part of the coupling section between the power unit 51 and insertion-section-side body 50. FIG. 12 is a transverse cross-sectional view of the part in the vicinity of the electric contacts 81. Solenoid fixing metal plates 90 are provided on both side portions of the casing 72. Self-hold type solenoids 91 of the above-described electromagnetic clutches are fixed to the solenoid fixing metal plates 90. The electromagnetic clutches are provided in the transmission systems between the deceleration gears 77 and the rotational shafts 78 of the driving unit 73a for UD and the driving unit 73b for RL. The electromagnetic clutches, which are provided with the self-hold type solenoids 91, have functions of selectively transmitting the driving force of the driving unit 73a for UD and the driving unit 73b for RL to the driving-side couplings 80.

Board fixing metal plates 92 and 93 are provided on upper and lower parts of the casing 72. A motor driving circuit board 94 is attached to the upper-side board fixing metal plate 92, and an image pick-up circuit board 95 is attached to the lower-side board fixing metal plate 93.

The electric contacts 81 are surrounded by the solenoid fixing metal plates 90 and board fixing metal plates 92 and 93. Thereby, electric signals for image pick-up, which are conducted via the electric contacts 81, are shielded from the outside of the power unit 51, and the influence of external noise can be prevented.

Next, the operation of the medical apparatus having the above-described structure is described. When the endoscope 21 is used, the insertion-section-side body 50 and the power unit 51 are detachably coupled. At this time, the insertion-section-side body 50 is connected to the reception chamber 71 of the power unit 51. The reception chamber 71 is provided with the driving-side couplings 80, electric contacts 81 and light guide connector receiving portion 82. The insertion-section-side body 50 is provided with the driven-side couplings 57, electrodes 59 and light guide connector 58.

If the insertion-section-side body 50 is connected to the reception chamber 71, the driven-side couplings 57 engage the driving-side couplings 80. Thereby, the rotation force of the driving-side couplings 80, which are driven by the electric motors 40, is transmitted to the bend-driving mechanism 38 via the driven-side couplings 57. Thus, the driving unit 73a for UD and the driving unit 73b for RL can be driven by motor driving.

In addition, when the insertion-section-side body 50 and reception chamber 71 are connected, the electrodes 59 of the insertion-section-side body 50 come in contact with the electric contacts 81 and are rendered electrically conductive. Thereby, electric signals relating to the image pick-up unit, such as a solid image pick-up element, can be transmitted/received via the first flexible board 67, connector 68, second flexible board 69 and image pick-up printed board 70.

Moreover, when the insertion-section-side body 50 and reception chamber 71 are connected, the light guide connector 58 of the insertion-section-side body 50 is connected to the light guide connector receiving portion 82. Thereby, the light source device 14 and the light guide 46 are optically connected, and illumination light can be guided to the distal-end structure section 26 of the endoscope 21.

The following advantageous effects can be obtained by the above-described structure. Specifically, in the present embodiment, when the insertion-section-side body 50 and the reception chamber 71 of the power unit 51 are connected, the non-fluid connection unit is constituted by the coupling section between the driving-side couplings 80 and driven-side couplings 57, the connection section between the electric contacts 81 and electrodes 59, and the connection section between the light guide connector receiving portion 82 and light guide connector 58. This non-fluid connection unit is disposed within the casing 72 of the power unit 51. Further, the fluid connection connector 84 is received in the recess portion 83 under the lower surface of the casing 72, and the fluid connection unit is constituted such that fluid connection connectors of air-feed, water-feed and forward water-feed conduits are connected outside the power unit 51. Thus, the non-fluid connection unit for the optical, electrical and driving force systems is provided within the reception chamber 71 that is surrounded by the peripheral walls, and the fluid connection unit for the air-feed, water-feed and forward water-feed conduits is provided outside the reception chamber 71. Thus, fluid connection can be shut off from non-fluid connection. Thereby, no fluid enters the non-fluid connection unit for the optical, electrical and driving force systems, and the reliability can be enhanced. As a result, it is possible to provide a medical apparatus such as an endoscope, wherein the fluid connection unit for fluid connection of, e.g. air feed and water feed, can be shut off, by the simple structure, from the non-fluid connection unit for non-fluid connection of, e.g. the illumination optical system, signal lines and driving force system, and the reliability of water-proof countermeasures is high.

The insertion-section-side body 50 is provided with the angular cylindrical portion 55 and circular cylindrical portion 56. The reception chamber 71 is provided with the angular cylindrical hole portion 71x and circular cylindrical hole portion 71y. When the insertion-section-side body 50 and reception chamber 71 are connected, the angular cylindrical portion 55 and angular cylindrical hole portion 71x are engaged and at the same time the circular cylindrical portion 56 and circular cylindrical hole portion 71y are engaged. Accordingly, by connecting the insertion-section-side body 50 to the reception chamber 71, the positioning of the insertion-section-side body 50 in the alignment direction with the power unit 51 can be carried out. Moreover, even if external force acts on the insertion-section-side body 50 during the use of the endoscope 21, the insertion-section-side body 50 can be held with the rigidity of the power unit 51 and the durability is high.

Besides, if the insertion-section-side body 50 is inserted in the reception chamber 71 in the state in which the fluid connection connector 84 is received in the recess portion 83 of the casing 72 of the power unit 51, the air-feed mouthpiece 53a, water-feed mouthpiece 53b and forward water-feed mouthpiece 53c of the insertion-section-side body are substantially simultaneously connected to the three connection ports 85 of the fluid connection connector 84. The three connection ports 85 of the fluid connection connector 84 are connected to the three tubes 33a, 33b and 33c for performing air feed, water feed and forward water feed. Thus, since the fluid connection is effected outside the power unit 51, even if liquid leak occurs from the fluid connection connector 84 due to the user's erroneous operation at the time of attaching/detaching the insertion-section-side body 50, there is no concern that liquid enters the non-fluid connection unit within the reception chamber 71 of the power unit 51, and the reliability can be enhanced. Furthermore, since all non-fluid connections are made within the reception chamber 71, the connectors of the optical, electrical and driving force systems are not exposed to the outside, and these connections do not interfere with other obstacles.

FIG. 13 shows a second embodiment of the invention. The structural parts common to those in the first embodiment are denoted by like reference numerals, and a description thereof is omitted here. The projection portion 52 on the outer peripheral surface of the cylindrical casing portion 202 of the insertion-section-side body 50 is provided with the forceps insertion hole 45 and a water leak detection mouthpiece 96 which neighbors the forceps insertion hole 45. The forceps insertion hole 45 is inclined outward, relative to the axial direction of the insertion-section-side body 50. The water leak detection mouthpiece 96 is disposed parallel to the forceps insertion hole 45.

According to the present embodiment, the operability of cleaning in the endoscope auto-cleaning device can be improved.

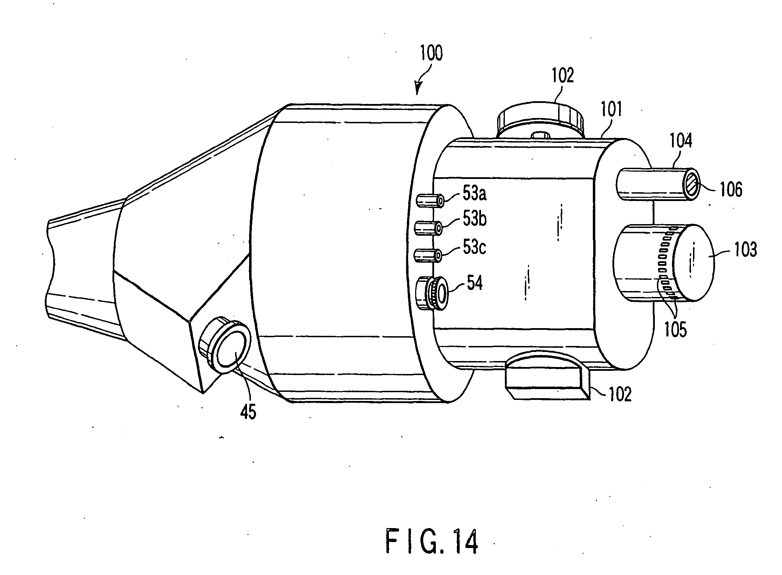

FIG. 14 shows an insertion-section-side body of an endoscope. The structural parts common to those in the first embodiment are denoted by like reference numerals, and a description thereof is omitted here. A proximal end portion of an insertion-section-side body 100 is provided with a modified-shaped cylindrical portion 101. The modified-shaped cylindrical portion 101 has arcuate surfaces and flat surfaces.

Driven-side couplings 102 are provided on the modified-shaped cylindrical portion 101. A first connection cylinder 103 of a circular cylindrical shape and a second connection cylinder 104 of a circular cylindrical shape are rearwardly projectingly provided on a rear end portion of the modified-shaped cylindrical portion 101 in such a manner that the first connection cylinder 103 and second connection cylinder 104 are parallel to each other. A great number of electrodes 105 are disposed on an outer peripheral surface of the first connection cylinder 103 at intervals in the circumferential direction. A light guide 106 is inserted in the second connection cylinder 104.

By inserting the insertion-section-side body 100 into the power unit (not shown), the first connection cylinder 103 and second connection cylinder 104 are connected at the same time. Thereby, electrical connection and optical connection, as well as driving power transmission, are effected.

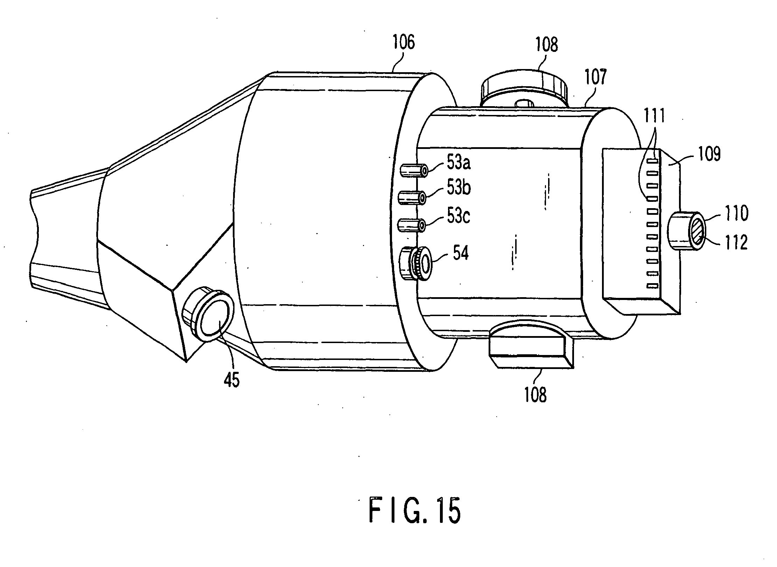

FIG. 15 shows an insertion-section-side body of an endoscope.

The structural parts common to those in the first embodiment are denoted by like reference numerals, and a description thereof is omitted here. A proximal end portion of an insertion-section-side body 100 is provided with a modified-shaped cylindrical portion 107 having arcuate surfaces and flat surfaces. Driven-side couplings 108 are provided on the modified-shaped cylindrical portion 107. An angular cylindrical portion 109 is provided on a rear end portion of the modified-shaped cylindrical portion 107. A circular cylindrical portion 110 is rearwardly projectingly provided on the angular cylindrical portion 109. A great number of electrodes 111 are juxtaposed on upper and lower surfaces of the angular cylindrical portion 109 at intervals in the width direction. A light guide 112 is inserted in the circular cylindrical portion 110.

By inserting the insertion-section-side body 100 into the power unit (not shown), the modified-shaped cylindrical portion 107 and circular cylindrical portion 110 are connected at the same time, and electrical connection and optical connection, as well as driving power transmission, are effected.

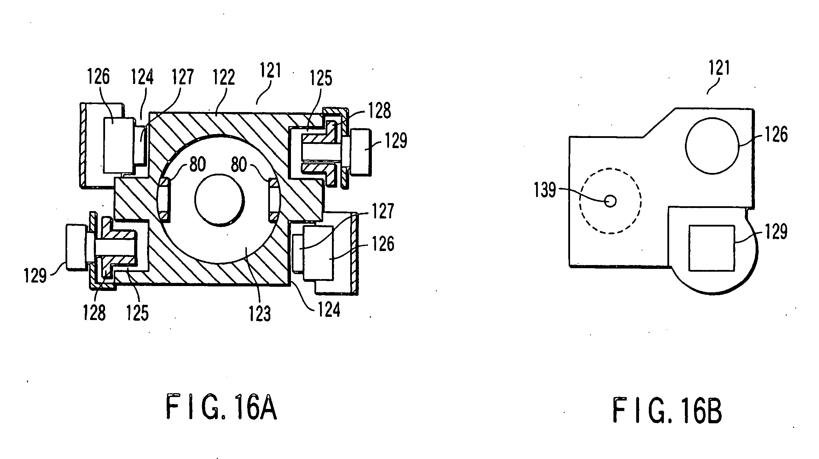

FIG. 16A and FIG. 16B show a further embodiment of the invention. FIG. 16A is a transverse cross-sectional front view showing the internal structure of a power unit 121, and FIG. 16B is a side view of the power unit 121. A casing 122 has a rectangular outside shape. A reception chamber 123 having a circular cross section is provided in the casing 122. First and second notch portions 124 and 125 are provided in dead spaces at upper and lower portions of both side parts of the casing 122. An electric motor 126 and an encoder 127 are received in the first notch portion 124. A deceleration gear 128 and a potentio-sensor 129 are received in the second notch portion 125.

As described above, the electric motor 126 with encoder 127 and the potentio-sensor 129 with deceleration gear 128 are disposed on upper and lower sides. The deceleration gear 128 is configured to transmit driving force to a rotational shaft 139 that is offset on the front side.

The present invention is not limited directly to the above-described embodiments. In practice, the structural elements can be modified. For example, some structural elements may be omitted from all the structural elements disclosed in the embodiments. Furthermore, structural elements in different embodiments may properly be combined.

## Claims

1. A medical apparatus comprising:
a first member (50) which constitutes a part of the medical apparatus;
a second member (51) having an outer peripheral portion which is surrounded by walls, and having a reception chamber (71) to which the first member (50) is adapted to be detachably connected;
a fluid connection unit (84) which is adapted to be connected to the first member (50) on an outside of the reception chamber (71);
a non-fluid connection unit which is adapted to be connected to the first member (50) on an inside of the reception chamber (71);
wherein the fluid connection unit (84) and the non-fluid connection unit are adapted to be connected at the same time when the first member (50) and the second member (51) are connected;
an angular cylindrical hole portion (71x) having a rectangular cross-sectional shape formed at a distal end side of the reception chamber (71), and
an angular cylindrical portion (55) with a rectangular cross-sectional shape provided on the first member (50) and adapted to be engaged in the angular cylindrical hole portion (71x) of the reception chamber (71);
a circular cylindrical hole portion (71y) having a circular cross-sectional shape formed on a depth side of the reception chamber (71); and
a circular cylindrical portion (56) with a circular cross-sectional shape provided on the first member (50) which is adapted to be engaged in the circular cylindrical hole portion (71y) of the reception chamber (71),
**characterised in that**
a light guide connector (58) extends from an end face of the circular cylindrical portion (56) of the first member, and
a light guide connector receiving portion (82) extends from an end face of the circular cylindrical hole portion (71y) of the reception chamber (71), wherein the light guide connector receiving portion (82) is adapted to be connected to the light guide connector (58) when the first member is inserted into the reception chamber (71), and
an outer peripheral surface of the circular cylindrical portion (56) is provided with electrodes (59), and an inner peripheral surface of the circular cylindrical hole portion (71y) is provided with electric contacts (81), wherein the electric contacts (81) are adapted to be put in contact with the electrodes (59) when the first member (50) is inserted into the reception chamber (71).

2. The medical apparatus according to claim 1, **characterized in that** the fluid connection unit is a fluid connection connector (84) of at least one of an air-feed conduit, a water-feed conduit and a forward water-feed conduit.

3. The medical apparatus according to claim 1, **characterized in that** the non-fluid connection unit is a connection connector of at least one of light, electricity and driving force.

4. The medical apparatus according to claim 1, **characterized in that** a driving-side member (80) for driving force transmission is provided on a side wall of the reception chamber (71), and the first member is provided with a driven-side member (57) which is adapted to be connected to the driving-side member (80) when the first member is inserted in the reception chamber (71).

5. The medical apparatus according to claim 1, **characterized in that** the first member is an endoscope body (50) having an insertion section (23) which is adapted to be inserted into a body cavity, and the second member is a power unit (51) which is adapted to execute control of an endoscope.

6. The medical apparatus according to claim 5, **characterized in that** the power unit (51) includes a connection section for connection to the endoscope body (50), to which at least one of an optical system, an electrical system and a driving force system is connected.

7. The medical apparatus according to claim 5, **characterized in that** the endoscope body (50) includes a fluid connection connector receiver (53a, 53b, 53c) of at least one of an air-feed conduit, a water-feed conduit and a forward water-feed conduit, and the power unit (51) is provided with a fluid connection connector (84) on an outside of the reception chamber (71), the fluid connection connector (84) being adapted to be connected to the fluid connection connector receiver (53a, 53b, 53c).

8. The medical apparatus according to claim 5, **characterized in that** the endoscope body (50) includes a bending section (25) which is provided in the insertion section (23), and a driven-side member (57) which is interlocked with the bending section (25), the power unit (51) includes, in the reception chamber (71) in which the endoscope body (50) is adapted to be received, a driving-side member (80) of a bend-driving mechanism which is driven by an electric motor (40), and the driving-side member (80) and the driven-side member (57) are adapted to be connected when the endoscope body (50) is received in the reception chamber (71).

9. The medical apparatus according to claim 5, **characterized in that** the endoscope body (50) includes a bending section (25) which is provided in the insertion section (23), and a driven-side member (57) which is interlocked with the bending section (25), the power unit (51) includes, in the reception chamber (71) in which the endoscope body (50) is adapted to be received, a driving-side member (80) which is driven by an operation handle, and the driving-side member (80) and the driven-side member (57) are adapted to be connected when the endoscope body (50) is received in the reception chamber (71).

## Patentansprüche

1. Medizinisches Gerät aufweisend:
ein erstes Element (50), das einen Teil des medizinischen Gerätes bildet;
ein zweites Element (51) mit einem von Wänden umgebenen äußeren Umfangsbereich und mit einer Aufnahmekammer (71), mit der das erste Element (50) lösbar verbindbar ist;
eine Fluidverbindungseinheit (84), die so ausgebildet ist, dass sie an dem ersten Element (50) an einer Außenseite der Aufnahmekammer (71) angeschlossen werden kann;
eine Nicht-Fluidverbindungseinheit, die so ausgebildet ist, dass sie an dem ersten Element (50) an einer Innenseite der Aufnahmekammer (71) angeschlossen werden kann;
wobei die Fluidverbindungseinheit (84) und die Nicht-Fluidverbindungseinheit so ausgebildet sind, dass sie im verbundenen Zustand des ersten Elements (50) und des zweiten Elements (51) gleichzeitig angeschlossen werden können;
ein eine rechteckige Querschnittsform aufweisender winkelzylindrischer Öffnungsbereich (71x), der an einer distalen Endseite der Aufnahmekammer (71) ausgebildet ist; und
ein eine rechteckige Querschnittsform aufweisender winkelzylindrischer Bereich (55), der an dem ersten Element (50) vorgesehen und ausgebildet ist zum Ineingriffbringen mit dem winkelzylindrischen Öffnungsbereich (71x) der Aufnahmekammer (71);
ein eine kreisförmige Querschnittsform aufweisender kreiszylindrischer Öffnungsbereich (71y), der auf einer Tiefenseite der Aufnahmekammer (71) ausgebildet ist; und
ein eine kreisförmige Querschnittsform aufweisender kreiszylindrischer Bereich (56), der an dem ersten Element (50) vorgesehen ist, zum Ineingriffbringen mit dem zylindrischen Öffnungsbereich (71y) der Aufnahmekammer (71);
**dadurch gekennzeichnet, dass** sich ein Lichtleiter-Anschlussteil (58) von einer Endfläche des kreiszylindrischen Bereichs (56) erstreckt, und
dass sich ein Aufnahmeabschnitt (82) für das Lichtleiter-Anschlussteil (58) von einer Endfläche des kreiszylindrischen Öffnungsbereichs (71y) der Aufnahmekammer (71) erstreckt, wobei der Aufnahmeabschnitt (82) für das Lichtleiter-Anschlussteil (58) so ausgebildet ist, dass er beim Einbringen des ersten Elements in die Aufnahmekammer (71) an das Lichtleiter-Anschlussteil (58) angeschlossen werden kann, und
dass eine äußere Umfangsfläche des kreiszylindrischen Bereichs (56) mit Elektroden (59) versehen ist, und eine innere Umfangsfläche des kreiszylindrischen Öffnungsbereichs (71y) mit elektrischen Kontakten (81) versehen ist, wobei die elektrischen Kontakte (81) ausgebildet sind zum Inkontaktbringen mit den Elektroden (59) wenn das erste Element (50) in die Aufnahmekammer (71) eingebracht wird.

2. Medizinisches Gerät nach Anspruch 1, **dadurch gekennzeichnet, dass** die Fluidverbindungseinheit ein Fluidverbindungsanschlussteil (84) einer Luftzuführleitung, einer Wasserzuführleitung und/oder einer Vorlauf-Wasserzuführleitung ist.

3. Medizinisches Gerät nach Anspruch 1, **dadurch gekennzeichnet, dass** die Nicht-Fluidverbindungseinheit ein Verbindungsanschlussteil für Licht, Elektrizität und/oder eine Antriebskraft ist.

4. Medizinisches Gerät nach Anspruch 1, **dadurch gekennzeichnet, dass** ein antriebsseitiges Element (80) zum Übertragen der Antriebskraft an einer Seitenwand der Aufnahmekammer (71) vorgesehen ist, und dass das erste Element mit einem abtriebsseitigen Element (57) versehen ist, das so ausgebildet ist, dass es beim Einbringen des ersten Elements in die Aufnahmekammer (71) an das antriebsseitige Element (80) angeschlossen werden kann.

5. Medizinisches Gerät nach Anspruch 1, **dadurch gekennzeichnet, dass** das erste Element ein Endoskopköper (50) ist, mit einem Einführbereich (23) ausgebildet zum Einführen in eine Körperhöhle, und dass das zweite Element ein Antriebsaggregat (51) ist, ausgebildet zum Ausführen von Endoskop-Steuerungsvorgängen.

6. Medizinisches Gerät nach Anspruch 5, **dadurch gekennzeichnet, dass** das Antriebsaggregat (51) einen Anschlussbereich zum Anschließen an den Endoskopköper (50) aufweist, an den mindestens eine optische Anordnung, eine elektrische Anordnung und/oder eine Antriebskraftanordnung angeschlossen ist.

7. Medizinisches Gerät nach Anspruch 5, **dadurch gekennzeichnet, dass** der Endoskopköper (50) eine Fluidverbindungsanschlussteil-Aufnahme (53a, 53b, 53c) einer Luftzuführleitung, einer Wasserzuführleitung und/oder einer Vorlauf-Wasserzuführleitung umfasst, und dass das Antriebsaggregat (51) ein auf der Außenseite der Aufnahmekammer (71) vorgesehenes Fluidverbindungsanschlussteil (84) aufweist, wobei das Fluidverbindungsanschlussteil (84) ausgebildet ist zum Verbinden mit der Fluidverbindungsanschlussteil-Aufnahme (53a, 53b, 53c).

8. Medizinisches Gerät nach Anspruch 5, **dadurch gekennzeichnet, dass** der Endoskopköper (50) einen in dem Einführbereich (23) vorgesehenen Biegebereich (25) und ein mit dem Biegebereich (25) verriegeltes abtriebsseitiges Element (57) umfasst, dass das Antriebsaggregat (51) in der Aufnahmekammer (71), die zum Aufnehmen des Endoskopköpers (50) ausgebildet ist, ein antriebsseitiges Element (80) einer von einem Elektromotor (40) angetriebenen Biege-Antriebseinrichtung aufweist, und dass das antriebsseitiges Element (80) und das abtriebsseitigen Element (57) so ausgebildet sind, dass sie bei Aufnahme des Endoskopköpers (50) in der Aufnahmekammer (71) miteinander verbunden werden können.

9. Medizinisches Gerät nach Anspruch 5, **dadurch gekennzeichnet, dass** der Endoskopköper (50) einen in dem Einführbereich (23) vorgesehenen Biegebereich (25) und ein mit dem Biegebereich (25) verriegeltes abtriebsseitigen Element (57) umfasst, dass das Antriebsaggregat (51) in der Aufnahmekammer (71), die zum Aufnehmen des Endoskopköpers (50) ausgebildet ist, ein von einem Betätigungsgriff angetriebenes antriebsseitiges Element (80) aufweist, und dass das antriebsseitiges Element (80) und das abtriebsseitigen Element (57) so ausgebildet sind, dass sie bei Aufnahme des Endoskopköpers (50) in der Aufnahmekammer (71) miteinander verbunden werden können.

## Revendications

1. Appareil médical comprenant:
un premier élément (50) qui constitue une partie de l'appareil médical;
un second élément (51) comportant une partie périphérique extérieure qui est entourée par des parois, et comportant une chambre de réception (71) à laquelle le premier élément (50) est adapté pour être couplé de manière amovible ;
un module de raccordement de fluide (84) qui est adapté pour être raccordé avec le premier élément (50) sur une partie extérieure de la chambre de réception (71);
un module de connexion autre que de fluide qui est adapté pour être raccordé avec le premier élément (50) sur une partie intérieure de la chambre de réception (71);
dans lequel le module de raccordement de fluide (84) et le module de connexion autre que de fluide sont adaptés pour être raccordés en même temps lors du couplage du premier élément (50) et du second élément (51);
une partie de trou cylindrique angulaire (71x) ayant une forme de section transversale rectangulaire réalisée d'un côté d'extrémité distale de la chambre de réception (71), et
une partie cylindrique angulaire (55) ayant une forme de section transversale rectangulaire réalisée sur le premier élément (50) et adaptée pour être engagée dans la partie de trou cylindrique angulaire (71x) de la chambre de réception (71);
une partie de trou cylindrique circulaire (71y) ayant une forme de section transversale circulaire réalisée sur un côté de fond de la chambre de réception (71); et
une partie cylindrique circulaire (56) ayant une forme de section transversale circulaire, réalisée sur le premier élément (50), qui est adaptée pour être engagée dans la partie de trou cylindrique circulaire (71y) de la chambre de réception (71),
**caractérisé en ce que**:
un connecteur de guide optique (58) s'étend depuis une face d'extrémité de la partie cylindrique circulaire (56) du premier élément, et
une partie de réception de connecteur de guide optique (82) s'étend depuis une face d'extrémité de la partie de trou cylindrique circulaire (71y) de la chambre de réception (71), dans lequel la partie de réception de connecteur de guide optique (82) est adaptée pour être connectée avec le connecteur de guide optique (58) lorsque le premier élément est introduit dans la chambre de réception (71), et
une surface périphérique extérieure de la partie cylindrique circulaire (56) est pourvue d'électrodes (59), et une surface périphérique intérieure de la partie de trou cylindrique circulaire (71y) est pourvue de contacts électriques (81), dans lequel les contacts électriques (81) sont adaptés pour mise en contact avec les électrodes (59) lorsque le premier élément (50) est introduit dans la chambre de réception (71).

2. Appareil médical selon la revendication 1, **caractérisé en ce que** le module de raccordement de fluide est un raccord de raccordement de fluide (84) d'au moins l'un d'un conduit d'alimentation en air, d'un conduit d'alimentation en eau et d'un conduit d'alimentation en eau directe.

3. Appareil médical selon la revendication 1, **caractérisé en ce que** le module de connexion autre que de fluide est un connecteur de connexion de lumière, d'électricité et/ou d'une force d'entraînement.

4. Appareil médical selon la revendication 1, **caractérisé en ce qu'**un élément de côté entraînement (80) destiné à une transmission de force d'entraînement est réalisé sur une paroi latérale de la chambre de réception (71), et le premier élément est pourvu d'un élément de côté entraîné (57) qui est adapté pour être engagé avec l'élément de côté entraînement (80) lorsque le premier élément est introduit dans la chambre de réception (71).

5. Appareil médical selon la revendication 1, **caractérisé en ce que** le premier élément est un corps d'endoscope (50) comportant une section d'insertion (23) qui est adaptée pour être insérée dans une cavité corporelle, et le second élément est un module d'alimentation de puissance (51) qui est adapté pour exécuter une commande d'un endoscope.

6. Appareil médical selon la revendication 5, **caractérisé en ce que** le module d'alimentation de puissance (51) comprend une section de connexion prévue pour connexion avec le corps d'endoscope (50), à laquelle au moins l'un d'un système optique, d'un système électrique et d'un système de force d'entraînement est connecté.

7. Appareil médical selon la revendication 5, **caractérisé en ce que** le corps d'endoscope (50) comprend un récepteur de raccord de raccordement de fluide (53a, 53b, 53c) d'au moins l'un d'un conduit d'alimentation en air, d'un conduit d'alimentation en eau et d'un conduit d'alimentation en eau directe, et le module d'alimentation de puissance (51) est pourvu d'un raccord de raccordement de fluide (84) sur une partie extérieure de la chambre de réception (71), le raccord de raccordement de fluide (84) étant adapté pour être raccordé avec le récepteur de raccord de raccordement de fluide (53a, 53b, 53c).

8. Appareil médical selon la revendication 5, **caractérisé en ce que** le corps d'endoscope (50) comprend une section de fléchissement (25) qui est disposée dans la section d'insertion (23), et un élément de côté entraîné (57) qui est verrouillé mutuellement avec la section de fléchissement (25), le module d'alimentation de puissance (51) comprend, dans la chambre de réception (71) à laquelle le corps d'endoscope (50) est adapté pour réception, un élément de côté entraînement (80) d'un mécanisme de fléchissement - d'entraînement qui est entraîné par un moteur électrique (40), et l'élément de côté entraînement (80) et l'élément de côté entraîné (57) sont adaptés pour être en prise lorsque le corps d'endoscope (50) est reçu dans la chambre de réception (71).

9. Appareil médical selon la revendication 5, **caractérisé en ce que** le corps d'endoscope (50) comprend une section de fléchissement (25) qui est disposée dans la section d'insertion (23), et un élément de côté entraîné (57) qui est verrouillé mutuellement avec la section de fléchissement (25), le module d'alimentation de puissance (51) comprend, dans la chambre de réception à laquelle le corps d'endoscope (50) est adapté pour réception, un élément de côté entraînement (80) qui est entraîné par une poignée de manipulation, et l'élément de côté entraînement (80) et l'élément de côté entraîné (57) sont adaptés pour être en prise lorsque le corps d'endoscope (50) est reçu dans la chambre de réception (71).
